# EUROPEAN PATENT APPLICATION

(11) **EP 3 418 366 A1**
(43) Date of publication of application: **26.12.2018**
(21) Application number: 17176683.5
(22) Date of filing: 19.06.2017
(51) Int. Cl.: C11D 3/37, C11D 3/386, C11D 17/00

(54) **AUTOMATIC DISHWASHING CLEANING COMPOSITION**

(71) Applicant: The Procter & Gamble Company, Cincinnati, OH 45202 (US)
(72) Inventor: MARTIN, Rachel Elizabeth, Newcastle upon Tyne, NE12 9TS (GB); PRESTON, Karen Margaret, Newcastle upon Tyne, NE12 9TS (GB); SOUTER, Philip Frank, Newcastle upon Tyne, NE12 9TS (GB)
(74) Representative: Yorquez Ramirez, Maria Isabel

(57) **Abstract**

The present invention relates to a phosphate-free automatic dishwashing cleaning composition comprising a protease and an itaconic/sulfonic polymer comprising monomer units derived from styrenesulfonic acid and/or monomer units derived from 2-acrylamido-2-methylpropane-sulfonic acid.

## Description

### TECHNICAL FIELD

The present invention is in the field of cleaning. It relates to a cleaning product, in particular, it relates to a phosphate-free automatic dishwashing cleaning composition comprising a protease and an itaconic/sulfonic polymer. The composition provides good cleaning and finishing.

### BACKGROUND OF THE INVENTION

There is a permanent desire to improve the performance of automatic dishwashing compositions, their environmental profile and to reduce the energy required by the automatic dishwashing process.

Due to environmental concerns phosphate is being replaced by biodegradable complexing agents. These complexing agents are usually strong builders and can negatively affect the stability of enzymes, in particular the strong complexing agents can negatively affect proteases by extracting the central calcium metal ion of the active site of the protease. This effect can be more pronounced under overbuilt conditions, i.e., when a composition comprises high level of complexing agent, the load is not heavily soiled and/or the composition is used in soft water because there will be more free builder to complex with the central calcium metal ion of the active site of the protease thereby deactivating the protease.

The object of the present invention is to provide a phosphate-free automatic dishwashing composition that provides better cleaning under a plurality of conditions, including overbuilt conditions.

### SUMMARY OF THE INVENTION

According to the first aspect of the invention, there is provided a phosphate-free automatic dishwashing cleaning composition. By "phosphate-free" is herein understood that the composition comprises less than 1 %, preferably less than 0.1 % by weight of the composition of phosphate. The composition comprises a protease and an itaconic/sulfonic polymer. The itaconic/sulfonic polymer comprises monomer units derived from styrenesulfonic acid and/or monomer units derived from 2-acrylamido-2-methylpropane-sulfonic acid. The term "acid" herein encompasses salts thereof.

The composition provides good cleaning and good finishing, including lack of filming and spotting and provides good shine, in particular, on glass. It also provides good care specially by avoiding the formation of a coloured film on stainless steel items. The composition is environmentally friendly due to the biodegradability of the polymer. The composition of the invention can comprise builders and complexing agents in addition to the polymer.

Without wishing to be bound by theory, it is believed that the size and stearic configuration of the polymer precludes its interaction with the protease thereby maintaining the stability of the protease in the composition and during the wash.

By "itaconic/sulfonic polymer" is herein meant a polymer comprising monomer units derived from itaconic acid and monomer units derived from styrenesulfonic acid and/or monomer units derived from 2-acrylamido-2-methylpropane-sulfonic acid. The "itaconic/sulfonic polymer" is sometimes herein referred to as "the polymer" of the invention.

Preferably the polymer has a weight average molecular weight of from about 500 g/mole to 10,000 g/mole, more preferably from 800 g/mole to 5,000 g/mole. It is also preferred that the polymer is free of tri-substituted vinyl monomer impurities, such as citraconic acid and/or mesaconic acid isomers. This can provide improved chelating capabilities.

Preferred polymers for use herein comprise from 5% to 20% by weight thereof of monomer units derived from styrenesulfonic acid. Preferably the polymer has a weight average molecular weight of from about 800 g/mole to 10,000 g/mole.

Other preferred polymers for use herein comprise from 15% to 40% by weight thereof of monomer units derived from 2-acrylamido-2-methylpropane-sulfonic acid.

The composition preferably comprises an alkaline-metal carbonate. High levels of alkaline-metal carbonates have been found to be very effective in automatic dishwashing. A drawback associated with such high carbonate levels, however, is that calcium ions present in the washing water readily form precipitates with the carbonate that can give rise to filming and spotting. It has been surprisingly found that compositions in which the weight ratio of the alkaline-metal carbonate to the polymer is at least 31.1, preferably from at least 3:1 to about 8:1 are very good in terms of finishing.

### BRIEF DESCRIPTION OF THE DRAWINGS

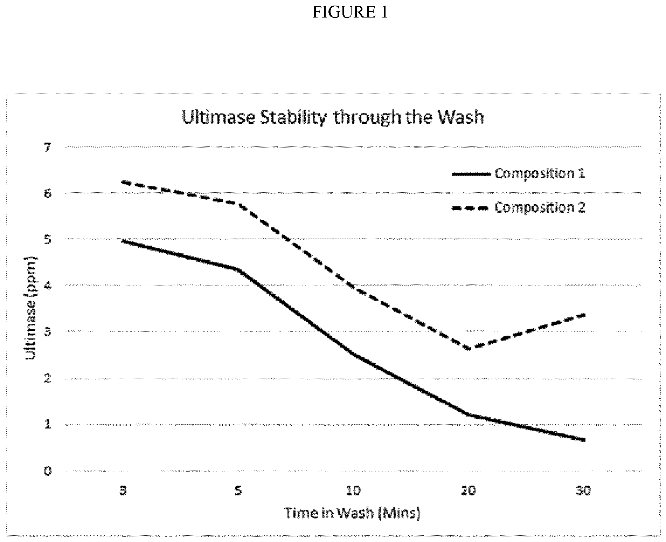

Figure 1 shows the stability of Ultimase® through the wash in two different compositions. Composition 1 (comparative) and Composition 2 (according to the invention).

### DETAILED DESCRIPTION OF THE INVENTION

The present invention encompasses an automatic dishwashing cleaning composition comprising a protease and a itaconic/sulfonic polymer comprising monomer units derived from styrenesulfonic acid and/or monomer units derived from 2-acrylamido-2-methylpropane-sulfonic acid. The composition provides very good cleaning and finishing, i.e, reduced filming, spotting and improved shine.

### Itaconic/sulfonic polymer

The polymer comprises monomer units derived from itaconic acid and monomer units derived from sulfonic acid, in particular monomer units derived from styrenesulfonic acid and/or 2-acrylamido-2-methylpropane-sulfonic acid. Preferably, the polymer is essentially free of tri-substituted vinyl monomer impurities.

Preferred polymers comprise styrene sulfonic acid, its salts or mixtures thereof. Even more preferably the polymer comprises from 5% to 20% by weight of the polymer of monomers comprising styrene sulfonic acid.

Preferably, the polymer is essentially free of tri-substituted vinyl monomer impurities. Preferably the polymer has a weight average molecular weight of from about 500 g/mole to about 10,000 g/mole, preferably from about 800 g/mole to about 5,000 g/mole. Preferably the polymer has a number average molecular weight of from about 500 g/mole to about 10,000 g/mole, preferably from about 800 g/mole to about 5,000 g/mole. Preferably the polymer has a polydispersity of about 2.0.

The polymer can be manufactured following the method of polymerization described in WO 2011/113069 A1. Especially preferred polymer for use herein, especially in combination with methyl glycine diacetic acid is Itaconix® provided by Itaconix.

Other preferred polymers comprise 2-acrylamido-2-methylpropane-sulfonic acid. Even more preferably the polymer comprises from 15% to 40% by weight of the polymer of monomer units derived from 2-acrylamido-2-methylpropane-sulfonic acid. Suitable polymers are described in WO2014/143773 A1 and WO2015/138872 A1 and supplied by Lubrizol.

### Proteases

In describing enzyme variants herein, the following nomenclature is used for ease of reference: Original amino acid(s):position(s):substituted amino acid(s). Standard enzyme IUPAC 1-letter codes for amino acids are used.

The composition of the invention is beneficial in terms of removal of proteinaceous soils.

Suitable proteases include metalloproteases and serine proteases, including neutral or alkaline microbial serine proteases, such as subtilisins (EC 3.4.21.62) as well as chemically or genetically modified mutants thereof. Suitable proteases include subtilisins (EC 3.4.21.62), including those derived from Bacillus, such as Bacillus lentus, B. alkalophilus, B. subtilis, B. amyloliquefaciens, Bacillus pumilus and Bacillus gibsonii.

Especially preferred proteases for the detergent of the invention are polypeptides demonstrating at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N87S, S99A, S99D, S99SD, S99SE, S101G, S101M, S103A, V104N/I, G118V, G118R, S128L, P129Q, S130A, Y167A, R170S, A194P, V205I, N218D and/or M222S, Q245R.

Most preferably the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) S9R + A15T + V68A + N218D+ Q245R;
(ii) G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G+V104N
(vii) S101M + G118V + S128L + P129Q + S130A

Suitable commercially available protease enzymes include those sold under the trade names Savinase®, Polarzyme®, Kannase®, Ovozyme®, Everlase®, Blaze®, Blaze Evity® and Esperase® by Novozymes A/S (Denmark), those sold under the tradename Properase®, Purafect®, Purafect Prime®, Purafect Ox®, FN3®, FN4®, Excellase®, Excellenz P®, Ultimase®, Eraser® and Purafect OXP® by Genencor International, those sold under the tradename Opticlean® and Optimase® by Solvay Enzymes, those available from Henkel/ Kemira, namely BLAP.

Preferred levels of protease in the composition of the invention include from about 0.2 to about 2 mg of active protease per grams of the composition.

### Automatic dishwashing cleaning composition

The automatic dishwashing cleaning composition can be in any physical form. It can be a loose powder, a gel or presented in unit dose form. Preferably it is in unit dose form, unit dose forms include pressed tablets and water-soluble packs. The automatic dishwashing cleaning composition of the invention is preferably presented in unit-dose form and it can be in any physical form including solid, liquid and gel form. The composition of the invention is very well suited to be presented in the form of a multi-compartment pack, more in particular a multi-compartment pack comprising compartments with compositions in different physical forms, for example a compartment comprising a composition in solid form and another compartment comprising a composition in liquid form. The composition is preferably enveloped by a water-soluble film such as polyvinyl alcohol. Especially preferred are compositions in unit dose form wrapped in a polyvinyl alcohol film having a thickness of less than 100 µm. The detergent composition of the invention weighs from about 8 to about 25 grams, preferably from about 10 to about 20 grams. This weight range fits comfortably in a dishwasher dispenser. Even though this range amounts to a low amount of detergent, the detergent has been formulated in a way that provides all the benefits mentioned herein above.

### Complexing agent

Preferably, the composition of the invention comprises at least 20%, more preferably at least 25%, especially from 25% to 50% by weight of the composition of the complexing agent.

A "complexing agent" is a compound capable of binding polyvalent ions such as calcium, magnesium, lead, copper, zinc, cadmium, mercury, manganese, iron, aluminium and other cationic polyvalent ions to form a water-soluble complex. The complexing agent has a logarithmic stability constant ([log K]) for Ca2+ of at least 5, preferably at least 6. The stability constant, log K, is measured in a solution of ionic strength of 0.1, at a temperature of 25° C.

The complexing agent is selected from the group consisting of citric acid, methyl-glycine-diacetic acid (MGDA), glutamic-N,N- diacetic acid (GLDA), iminodisuccinic acid (IDS), carboxy methyl inulin and mixtures thereof. Especially preferred complexing agent for use herein is MGDA, especially preferred for use herein is the three-sodium salt of MGDA. Preferably, the complexing agent is the three-sodium salt of MGDA and the polymer comprises monomer units derived from styrenesulfonic acid and/or salts thereof.

### Bleach

The composition of the invention preferably comprises from about 1 to about 20%, more preferably from about 5 to about 18%, even more preferably from about 8 to about 15% of bleach by weight of the composition.

Inorganic and organic bleaches are suitable for use herein. Inorganic bleaches include perhydrate salts such as perborate, percarbonate, persulfate and persilicate salts. The inorganic perhydrate salts are normally the alkali metal salts. The inorganic perhydrate salt may be included as the crystalline solid without additional protection. Alternatively, the salt can be coated. Suitable coatings include sodium sulphate, sodium carbonate, sodium silicate and mixtures thereof. Said coatings can be applied as a mixture applied to the surface or sequentially in layers.

Alkali metal percarbonates, particularly sodium percarbonate is the preferred bleach for use herein. The percarbonate is most preferably incorporated into the products in a coated form which provides in-product stability.
Potassium peroxymonopersulfate is another inorganic perhydrate salt of utility herein.

Typical organic bleaches are organic peroxyacids, especially dodecanediperoxoic acid, tetradecanediperoxoic acid, and hexadecanediperoxoic acid. Mono- and diperazelaic acid, mono- and diperbrassylic acid are also suitable herein. Diacyl and Tetraacylperoxides, for instance dibenzoyl peroxide and dilauroyl peroxide, are other organic peroxides that can be used in the context of this invention.

Further typical organic bleaches include the peroxyacids, particular examples being the alkylperoxy acids and the arylperoxy acids. Preferred representatives are (a) peroxybenzoic acid and its ring-substituted derivatives, such as alkylperoxybenzoic acids, but also peroxy-α-naphthoic acid and magnesium monoperphthalate, (b) the aliphatic or substituted aliphatic peroxy acids, such as peroxylauric acid, peroxystearic acid, ε-phthalimidoperoxycaproic acid[phthaloiminoperoxyhexanoic acid (PAP)], o-carboxybenzamidoperoxycaproic acid, N-nonenylamidoperadipic acid and N-nonenylamidopersuccinates, and (c) aliphatic and araliphatic peroxydicarboxylic acids, such as 1,12-diperoxycarboxylic acid, 1,9-diperoxyazelaic acid, diperoxysebacic acid, diperoxybrassylic acid, the diperoxyphthalic acids, 2-decyldiperoxybutane-1,4-dioic acid, N,N-terephthaloyldi(6-aminopercaproic acid).

### Bleach Activators

Bleach activators are typically organic peracid precursors that enhance the bleaching action in the course of cleaning at temperatures of 60° C and below. Bleach activators suitable for use herein include compounds which, under perhydrolysis conditions, give aliphatic peroxoycarboxylic acids having preferably from 1 to 12 carbon atoms, in particular from 2 to 10 carbon atoms, and/or optionally substituted perbenzoic acid. Suitable substances bear O-acyl and/or N-acyl groups of the number of carbon atoms specified and/or optionally substituted benzoyl groups. Preference is given to polyacylated alkylenediamines, in particular tetraacetylethylenediamine (TAED), acylated triazine derivatives, in particular 1,5-diacetyl-2,4-dioxohexahydro-1,3,5-triazine (DADHT), acylated glycolurils, in particular tetraacetylglycoluril (TAGU), N-acylimides, in particular N-nonanoylsuccinimide (NOSI), acylated phenolsulfonates, in particular n-nonanoyl- or isononanoyloxybenzenesulfonate (n- or iso-NOBS), decanoyloxybenzoic acid (DOBA), carboxylic anhydrides, in particular phthalic anhydride, acylated polyhydric alcohols, in particular triacetin, ethylene glycol diacetate and 2,5-diacetoxy-2,5-dihydrofuran and also triethylacetyl citrate (TEAC). If present the composition of the invention comprises from 0.01 to 5, preferably from 0.2 to 2% by weight of the composition of bleach activator, preferably TAED.

### Bleach Catalyst

The composition herein preferably contains a bleach catalyst, preferably a metal containing bleach catalyst. More preferably the metal containing bleach catalyst is a transition metal containing bleach catalyst, especially a manganese or cobalt-containing bleach catalyst.

Bleach catalysts preferred for use herein include manganese triazacyclononane and related complexes; Co, Cu, Mn and Fe bispyridylamine and related complexes; and pentamine acetate cobalt(III) and related complexes.

Preferably the composition of the invention comprises from 0.001 to 0.5, more preferably from 0.002 to 0.05% of bleach catalyst by weight of the composition. Preferably the bleach catalyst is a manganese bleach catalyst.

### Builders

The composition of the invention preferably comprises a builder. Suitable additional builders are selected from the group consisting of carbonate, silicate and mixtures thereof. Especially preferred for use herein is sodium carbonate. Preferably the composition of the invention comprises from 5 to 50%, more preferably from 10 to 40% and especially from 15 to 30% of sodium carbonate by weight of the composition. Preferably the composition is free of silicate.

### Other polymers

The composition of the invention can also comprise polymers free of itaconic monomers. Other polymers can be used in any suitable amount from about 0.1 to about 10%, preferably from 0.2 to about 15%, more preferably from 0.3 to 8% by weight of the composition

Other polymers include sulfonated derivatives of polycarboxylic acids and may comprise two, three, four or more different monomer units. The preferred copolymers contain:
At least one structural unit derived from a carboxylic acid monomer having the general formula (III): 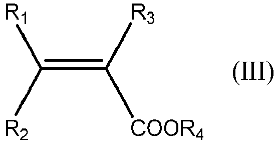 wherein R₁ to R₃ are independently selected from hydrogen, methyl, linear or branched saturated alkyl groups having from 2 to 12 carbon atoms, linear or branched mono or polyunsaturated alkenyl groups having from 2 to 12 carbon atoms, alkyl or alkenyl groups as aforementioned substituted with -NH2 or -OH, or -COOH, or COOR₄, where R₄ is selected from hydrogen, alkali metal, or a linear or branched, saturated or unsaturated alkyl or alkenyl group with 2 to 12 carbons;
Preferred carboxylic acid monomers include one or more of the following: acrylic acid, maleic acid, maleic anhydride, citraconic acid, 2-phenylacrylic acid, cinnamic acid, crotonic acid, fumaric acid, methacrylic acid, 2-ethylacrylic acid, methylenemalonic acid, or sorbic acid. Acrylic and methacrylic acids being more preferred.

Optionally, one or more structural units derived from at least one nonionic monomer having the general formula (IV):

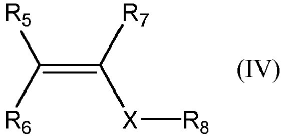

Wherein R₅ to R₇ are independently selected from hydrogen, methyl, phenyl or hydroxyalkyl groups containing 1 to 6 carbon atoms, and can be part of a cyclic structure, X is an optionally present spacer group which is selected from -CH₂-, -COO-, -CONH- or -CONR₈-, and R₈ is selected from linear or branched, saturated alkyl radicals having 1 to 22 carbon atoms or unsaturated, preferably aromatic, radicals having from 6 to 22 carbon atoms.

Preferred non-ionic monomers include one or more of the following: butene, isobutene, pentene, 2-methylpent-1-ene, 3-methylpent-1-ene, 2,4,4-trimethylpent-1-ene, 2,4,4-trimethylpent-2-ene, cyclopentene, methylcyclopentene, 2-methyl-3-methyl-cyclopentene, hexene, 2,3-dimethylhex-1-ene, 2,4-dimethylhex-1-ene, 2,5-dimethylhex-1-ene, 3,5-dimethylhex-1-ene, 4,4-dimethylhex-1-ene, cyclohexene, methylcyclohexene, cycloheptene, alpha olefins having 10 or more carbon atoms such as, dec-1-ene, dodec-1-ene, hexadec-1-ene, octadec-1-ene and docos-1-ene, preferred aromatic monomers are styrene, alpha methylstyrene, 3-methylstyrene, 4-dodecylstyrene, 2-ethyl-4-bezylstyrene, 4-cyclohexylstyrene, 4-propylstyrol, 1-vinylnaphtalene, 2-vinylnaphtalene; preferred carboxylic ester monomers are methyl (meth)acrylate, ethyl (meth)acrylate, propyl (meth)acrylate, t-butyl (meth)acrylate, pentyl (meth)acrylate, hexyl (meth)acrylate, 2-ethylhexyl (meth)acrylate, octyl (meth)acrylate, lauryl (meth)acrylate, stearyl (meth)acrylate and behenyl (meth)acrylate; preferred amides are N-methyl acrylamide, N-ethyl acrylamide, N-t-butyl acrylamide, N-2-ethylhexyl acrylamide, N-octyl acrylamide, N-lauryl acrylamide, N-stearyl acrylamide, N-behenyl acrylamide.

And at least one structural unit derived from at least one sulfonic acid monomer having the general formula (V) and (VI): 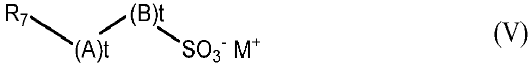 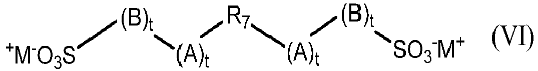 wherein R₇ is a group comprising at least one sp2 bond, A is O, N, P, S, an amido or ester linkage, B is a mono- or polycyclic aromatic group or an aliphatic group, each t is independently 0 or 1, and M+ is a cation. In one aspect, R₇ is a C2 to C6 alkene. In another aspect, R7 is ethene, butene or propene.

Preferred sulfonated monomers include one or more of the following: 1-acrylamido-1-propanesulfonic acid, 2-acrylamido-2-propanesulfonic acid, 2-acrylamido-2-methyl-1-propanesulfonic acid, 2-methacrylamido-2-methyl-1-propanesulfonic acid, 3- methacrylamido-2-hydroxy-propanesulfonic acid, allylsulfonic acid, methallylsulfonic acid, allyloxybenzenesulfonic acid, methallyloxybenzenesulfonic acid, 2-hydroxy-3- (2-propenyloxy) propanesulfonic acid, 2-methyl-2-propen-1-sulfonic acid, styrenesulfonic acid, vinylsulfonic acid, 3-sulfopropyl, 3-sulfo-propylmethacrylate, sulfomethacrylamide, sulfomethylmethacrylamide and mixtures of said acids or their water-soluble salts.

Preferably, the polymer comprises the following levels of monomers: from about 40 to about 90%, preferably from about 60 to about 90% by weight of the polymer of one or more carboxylic acid monomer; from about 5 to about 50%, preferably from about 10 to about 40% by weight of the polymer of one or more sulfonic acid monomer; and optionally from about 1% to about 30%, preferably from about 2 to about 20% by weight of the polymer of one or more non-ionic monomer. An especially preferred polymer comprises about 70% to about 80% by weight of the polymer of at least one carboxylic acid monomer and from about 20% to about 30% by weight of the polymer of at least one sulfonic acid monomer.

In the polymers, all or some of the carboxylic or sulfonic acid groups can be present in neutralized form, i.e. the acidic hydrogen atom of the carboxylic and/or sulfonic acid group in some or all acid groups can be replaced with metal ions, preferably alkali metal ions and in particular with sodium ions.

The carboxylic acid is preferably (meth)acrylic acid. The sulfonic acid monomer is preferably 2-acrylamido-2-propanesulfonic acid (AMPS).

Preferred commercial available polymers include: Alcosperse 240, Aquatreat AR 540 and Aquatreat MPS supplied by Alco Chemical; Acumer 3100, Acumer 2000, Acusol 587G and Acusol 588G supplied by Rohm & Haas; Goodrich K-798, K-775 and K-797 supplied by BF Goodrich; and ACP 1042 supplied by ISP technologies Inc. Particularly preferred polymers are Acusol 587G and Acusol 588G supplied by Rohm & Haas.

Other polymers include anionic carboxylic polymer of low molecular weight. They can be homopolymers or copolymers with a weight average molecular weight of less than or equal to about 200,000 g/mol, or less than or equal to about 75,000 g/mol, or less than or equal to about 50,000 g/mol, or from about 3,000 to about 50,000 g/mol, preferably from about 5,000 to about 45,000 g/mol. The dispersant polymer may be a low molecular weight homopolymer of polyacrylate, with an average molecular weight of from 1,000 to 20,000, particularly from 2,000 to 10,000, and particularly preferably from 3,000 to 5,000.

The other polymer may be a copolymer of acrylic with methacrylic acid, acrylic and/or methacrylic with maleic acid, and acrylic and/or methacrylic with fumaric acid, with a molecular weight of less than 70,000. Their molecular weight ranges from 2,000 to 80,000 and more preferably from 20,000 to 50,000 and in particular 30,000 to 40,000 g/mol. and a ratio of (meth)acrylate to maleate or fumarate segments of from 30:1 to 1:2.

The other polymer may be a copolymer of acrylamide and acrylate having a molecular weight of from 3,000 to 100,000, alternatively from 4,000 to 20,000, and an acrylamide content of less than 50%, alternatively less than 20%, by weight of the dispersant polymer can also be used. Alternatively, such dispersant polymer may have a molecular weight of from 4,000 to 20,000 and an acrylamide content of from 0% to 15%, by weight of the polymer.

Alternatively, the other polymer can be selected from the group consisting of alkoxylated polyalkyleneimines, alkoxylated polycarboxylates, polyethylene glycols, styrene co-polymers, cellulose sulfate esters, carboxylated polysaccharides, amphiphilic graft copolymers and mixtures thereof.

The composition of the invention can be free of other polymers.

### Surfactant

Surfactants suitable for use herein include non-ionic surfactants, preferably the compositions are free of any other surfactants. Traditionally, non-ionic surfactants have been used in automatic dishwashing for surface modification purposes in particular for sheeting to avoid filming and spotting and to improve shine. It has been found that non-ionic surfactants can also contribute to prevent redeposition of soils.

Preferably the composition of the invention comprises a non-ionic surfactant or a non-ionic surfactant system, more preferably the non-ionic surfactant or a non-ionic surfactant system has a phase inversion temperature, as measured at a concentration of 1% in distilled water, between 40 and 70°C, preferably between 45 and 65°C. By a "non-ionic surfactant system" is meant herein a mixture of two or more non-ionic surfactants. Preferred for use herein are non-ionic surfactant systems. They seem to have improved cleaning and finishing properties and better stability in product than single non-ionic surfactants.

Phase inversion temperature is the temperature below which a surfactant, or a mixture thereof, partitions preferentially into the water phase as oil-swollen micelles and above which it partitions preferentially into the oil phase as water swollen inverted micelles. Phase inversion temperature can be determined visually by identifying at which temperature cloudiness occurs.

The phase inversion temperature of a non-ionic surfactant or system can be determined as follows: a solution containing 1% of the corresponding surfactant or mixture by weight of the solution in distilled water is prepared. The solution is stirred gently before phase inversion temperature analysis to ensure that the process occurs in chemical equilibrium. The phase inversion temperature is taken in a thermostable bath by immersing the solutions in 75 mm sealed glass test tube. To ensure the absence of leakage, the test tube is weighed before and after phase inversion temperature measurement. The temperature is gradually increased at a rate of less than 1°C per minute, until the temperature reaches a few degrees below the pre-estimated phase inversion temperature. Phase inversion temperature is determined visually at the first sign of turbidity.

Suitable nonionic surfactants include: i) ethoxylated non-ionic surfactants prepared by the reaction of a monohydroxy alkanol or alkyphenol with 6 to 20 carbon atoms with preferably at least 12 moles particularly preferred at least 16 moles, and still more preferred at least 20 moles of ethylene oxide per mole of alcohol or alkylphenol; ii) alcohol alkoxylated surfactants having a from 6 to 20 carbon atoms and at least one ethoxy and propoxy group. Preferred for use herein are mixtures of surfactants i) and ii).

Another suitable non-ionic surfactants are epoxy-capped poly(oxyalkylated) alcohols represented by the formula:

R1O[CH2CH(CH3)O]x[CH2CH2O]y[CH2CH(OH)R2] (I)

wherein R1 is a linear or branched, aliphatic hydrocarbon radical having from 4 to 18 carbon atoms; R2 is a linear or branched aliphatic hydrocarbon radical having from 2 to 26 carbon atoms; x is an integer having an average value of from 0.5 to 1.5, more preferably about 1; and y is an integer having a value of at least 15, more preferably at least 20.

Preferably, the surfactant of formula I, at least about 10 carbon atoms in the terminal epoxide unit [CH2CH(OH)R2]. Suitable surfactants of formula I, according to the present invention, are Olin Corporation's POLY-TERGENT® SLF-18B nonionic surfactants, as described, for example, in WO 94/22800, published October 13, 1994 by Olin Corporation.

### Amylases

The composition of the invention can comprise amylases. A preferred alkaline amylase is derived from a strain of Bacillus, such as Bacillus licheniformis, Bacillus amyloliquefaciens, Bacillus stearothermophilus, Bacillus subtilis, or other Bacillus sp., such as Bacillus sp. NCIB 12289, NCIB 12512, NCIB 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) the variants described in US 5,856,164 and WO99/23211, WO 96/23873, WO00/60060 and WO 06/002643, especially the variants with one or more substitutions in the following positions versus the AA560 SEQ ID No. 3: 9, 26, 30, 33, 82, 37, 106, 118, 128, 133, 149, 150, 160, 178, 182, 186, 193, 195, 202, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482, 484, preferably that also contain the deletions of D183* and G184*.
(b) variants exhibiting at least 95% identity with the wild-type enzyme from Bacillus sp.707 (SEQ ID NO:7 in US 6,093, 562), especially those comprising one or more of the following mutations M202, M208, S255, R172, and/or M261. Preferably said amylase comprises one of M202L or M202T mutations.
(c) variants exhibiting at least 95% identity with SEQ ID NO:1 of WO2016091688, especially those comprising one or more, preferably two or more or even three or more of the following mutations H183*, G184*, I405L, A421H, A422P and A428T.

Suitable commercially available alpha-amylases include DURAMYL®, LIQUEZYME®, TERMAMYL®, TERMAMYL ULTRA®, NATALASE®, EVEREST®, SUPRAMYL®, STAINZYME®, STAINZYME PLUS®, AMPLIFY®, FUNGAMYL® and BAN® (Novozymes A/S, Bagsvaerd, Denmark), KEMZYM® AT 9000 Biozym Biotech Trading GmbH Wehlistrasse 27b A-1200 Wien Austria, RAPIDASE® , PURASTAR®, ENZYSIZE®, OPTISIZE HT PLUS®, POWERASE®, EXCELLENZ™ S series, including EXCELLENZ™ S 1000 and EXCELLENZ™ S 2000 and PURASTAR OXAM® (DuPont Industrial Biosciences., Palo Alto, California) and KAM® (Kao, 14-10 Nihonbashi Kayabacho, 1-chome, Chuo-ku Tokyo 103-8210, Japan). Amylases especially preferred for use herein include AMPLIFY®, STAINZYME®, STAINZYME PLUS®, EXCELLENZ™ S 1000, EXCELLENZ™ S2000 and mixtures thereof.

Preferably, the composition of the invention comprises at least 0.005 mg, preferably from about 0.0025 to about 0.025, more preferably from about 0.05 to about 0.3, especially from about 0.01 to about 0.25 mg of active amylase.

Preferably, the protease and/or amylase of the composition of the invention are in the form of granulates, the granulates comprise more than 29% of sodium sulfate by weight of the granulate and/or the sodium sulfate and the active enzyme (protease and/or amylase) are in a weight ratio of between 3:1 and 100:1 or preferably between 4:1 and 30:1 or more preferably between 5:1 and 20:1.

### Crystal growth inhibitor

Crystal growth inhibitors are materials that can bind to calcium carbonate crystals and prevent further growth of species such as aragonite and calcite.

Especially preferred crystal growth inhibitor for use herein is HEDP (1-hydroxyethylidene 1,1-diphosphonic acid). Preferably, the composition of the invention comprises from 0.01 to 5%, more preferably from 0.05 to 3% and especially from 0.5 to 2% of a crystal growth inhibitor by weight of the product, preferably HEDP.

### Metal Care Agents

Metal care agents may prevent or reduce the tarnishing, corrosion or oxidation of metals, including aluminium, stainless steel and non-ferrous metals, such as silver and copper. Preferably the composition of the invention comprises from 0.1 to 5%, more preferably from 0.2 to 4% and especially from 0.3 to 3% by weight of the product of a metal care agent, preferably the metal care agent is benzo triazole (BTA).

### Glass Care Agents

Glass care agents protect the appearance of glass items during the dishwashing process. Preferably the composition of the invention comprises from 0.1 to 5%, more preferably from 0.2 to 4% and specially from 0.3 to 3% by weight of the composition of a metal care agent, preferably the glass care agent is a zinc containing material, specially hydrozincite.

The automatic dishwashing composition of the invention preferably has a pH as measured in 1% weight/volume aqueous solution in distilled water at 25°C of greater than 10.5, more preferably from about 10.6 to about 12, more preferably from about 10.7 to less than about 11.8 and especially from about 10.7 to about 11.5.

The automatic dishwashing composition of the invention preferably has a reserve alkalinity of from about 10 to about 20, more preferably from about 12 to about 18 at a pH of 9.5 as measured in NaOH with 100 grams of product at 20°C.

A preferred automatic dishwashing composition of the invention comprises:
i) from 2 to 20% by weight of the composition of bleach, preferably sodium percarbonate;
ii) from 10 to 40% by weight of the composition of sodium carbonate;
iii) from 10 to 40% by weight of the composition of MGDA;
iv) from 2 to 10% by weight of the composition of a non-ionic surfactant;
v) optionally but preferably a bleach catalyst, more preferably a manganese bleach catalyst.

Other optional ingredients include: a crystal growth inhibitor, preferably HEDP, and glass care agents.

### EXAMPLES

Two automatic dishwashing formulas were made comprising the ingredients detailed herein below (Composition 1 (comparative) and Composition 2 (according to the invention)).

| Ingredients (active grams) | Composition 1 | Composition 2 |
|---|---|---|
| Solid | | |
| MGDA | 5g | - |
| Itaconic/sulfonic polymer | - | 4g |
| Other sulphonated polymer | 0.4g | 0.4g |
| Sodium carbonate | 7g | 7g |
| Amylase | 8mg | 8 mg |
| Protease | 38mg | 38mg |
| Sodium percarbonate | 3g | 3g |
| Bleach catalyst and bleach activator | 4mg | 4mg |
| HEDP | 0.1g | 0.1g |
| Miscellaneous | 0.2g | 0.2g |

| **Liquid** | | |
|---|---|---|
| Plurafac SLF-180 | 0.8g | 0.8g |
| Lutensol TO7 | 0.9g | 0.9g |
| Miscellanous | 0.5g | 0.5g |
| **Calcium Binding Capacity of Formula (mg Ca²⁺/L)** | 160 | 163 |

| | |
|---|---|
| MGDA | Tri-sodium salt of methyl glycine diacetic acid |
| Other sulphonated polymer | Acusol 588 supplied by Dow |
| Protease | Ultimase ® available from DuPont |
| Bleach activator | TAED (Tetraacetylethylenediamine) |
| Bleach catalyst | Manganese bleach catalyst |
| HEDP | 1-hydroxyethylidene 1,1-diphosphonic acid |
| Plurafac SLF-180 | Nonionic surfactant supplied by BASF |
| Lutensol TO7 | Nonionic surfactant supplied by BASF |

### Protease Stability

Protease retention is measured through activity using N-Succinyl-ALA-ALA-PRO-PHE p-nitroanilide (PNA) as substrate. The mechanism for this chemistry is as follows: A solution of detergent/protease is introduced to the PNA substrate in solution. The enzyme cleaves bonds between amino acids and most importantly the amide bond between the phenylalanine and the p-nitroanilide group liberating p-nitroaniline, thus producing a yellow colour. The intensity of the colour (405nm) is proportional to the amount of enzyme in the solution.

Composition 1 and 2 were each solubilised in 5L of water at 50°C. The water hardness was 55ppm (Ca²⁺, Mg²⁺). The protease stability was measured over the course of 30 minutes for both compositions.

As it can be seen in Figure 1, in Composition 2 the level of protease is maintained higher than that of Composition 1 for the full 30 minutes of the experiment, indicating superior stability.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A phosphate-free automatic dishwashing cleaning composition comprising a protease and an itaconic/sulfonic polymer comprising monomer units derived from styrenesulfonic acid and/or monomer units derived from 2-acrylamido-2-methylpropane-sulfonic acid.

2. A composition according to claim 1 wherein the polymer comprises from 5% to 20% by weight thereof of monomer units derived from styrenesulfonic acid.

3. A composition according to the preceding claim wherein the polymer has a weight average molecular weight of from about 500 g/mole to 10,000 g/mole.

4. A composition according to claim 1 wherein the polymer comprises from 15% to 40% by weight thereof of monomer units derived from 2-acrylamido-2-methylpropane-sulfonic acid.

5. A composition according to any of the preceding claims wherein the polymer is essentially free of tri-substituted vinyl monomer impurities.

6. A composition according to any of the preceding claims wherein the composition comprises from 1 to 40% by weight of the composition of the polymer.

7. A composition according to any of the preceding claims further comprising a complexing agent.

8. A composition according to the preceding claim wherein the complexing agent is selected from the group consisting of citric acid, its salts and derivatives thereof, methyl glycine diacetic acid, its salts and derivatives thereof, glutamic-N,N- diacetic acid, its salts and derivatives thereof, iminodisuccinic acid, its salts and derivatives thereof carboxy methyl inulin, its salts and derivatives thereof

9. A composition according to any of claims 7 or 8 wherein the composition comprises from 10 to 50% by weight of the composition of complexing agent.

10. A composition according to any of the preceding claims wherein the composition has a pH greater than 10.5 as measured in a 1% by weight aqueous solution at 25°C.

11. A composition according to any of the preceding claims further comprising an alkaline-metal carbonate.

12. A composition according to the preceding claim wherein the weight ratio of the alkaline-metal carbonate to the polymer is at least 3:1.

13. A composition according to any of the preceding claims wherein the protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) S9R + A15T + V68A + N218D+ Q245R;
(ii) G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S101M + G118V + S128L + P129Q + S130A

14. A composition according to any of the preceding claims further comprising bleach.

15. A composition according to any of the preceding claims further comprising a crystal growth inhibitor.
